(19) Europäisches Patentamt
European Patent Office
Office européen des brevets

(11) Publication number: **0 450 508 A2**

# EUROPEAN PATENT APPLICATION

(21) Application number: 91104966.6

(22) Date of filing: 28.03.91

(51) Int. Cl.⁵: **A61K 31/73**, A61K 31/725

(30) Priority: 03.04.90 IT 1992490

(43) Date of publication of application:
09.10.91 Bulletin 91/41

(84) Designated Contracting States:
AT BE CH DE DK ES FR GB GR IT LI LU NL SE

(71) Applicant: MEDIOLANUM FARMACEUTICI s.r.l.
Via S.G. Cottolengo, 31
I-20143 Milano(IT)

(72) Inventor: Del Bono, Rinaldo
Via Mozart, 2
I-20122 Milan(IT)
Inventor: Ferrari, Gianni
Via Passarella, 4
I-20122 Milan(IT)

(74) Representative: Gervasi, Gemma et al
NOTARBARTOLO & GERVASI Srl Viale
Bianca Maria 33
I-20122 Milan(IT)

(54) **Use of dermatan sulphate for the prevention and therapy of cerebral aging and of central nervous system functional deficit states.**

(57) The present invention refers to the use of Dermatan sulphate, in its natural form and in its low molecular weight form, possibly in association with heparin, for the preparation of pharmaceutical compositions to be administered per os and parenterally in the preventive and therapeutic treatment of cerebral aging, of demential infantile and senile forms, of syndroms correlated to an altered biochemistry of the glycosaminoglycane sulphates, of hypofunctionality and dismetabolism of the central nervous system following an excessive use of drugs and to toxicodependency.

Prior art

Numerous experimental pharmacology and pathological istoanatomy studies have shown that in the mammal brain all the glycosaminoglycane sulphates are present (condroitin sulphate, heparan sulphate, heparin and jaluronic acid), with the exception of Keratan sulphate (H.G. Jenkins and H.S. Bachelard, J. Neurochem., 1988, 51(5), 1634).

The degree of biosynthesis and metabolizing, at the cerebral level, is different for the single glycosaminoglycane sulphates and is strictly correlated with the age and therefore with the degree of maturity of the brain (T. Burkart and U.N. Wiesmann, Dev. Biol. 1987, 120(2), 447).

Studies performed on rat fetal brain have evidenced, at this stage of maturity, significant levels of jaluronic acid and, at the start of development, also of heparan sulphate.

It was found furthermore that, in the rat brain the weight ratio between jaluronic acid and condroitin sulphate is approximately 1:1, starting from the 10th day after birth up to the 18th month and increases with the age up to approximate values of 2.6:1 (H.G. Jenkins and H.S. Bachelard, J. Neurochem., 1988, 51(5), 1634).

Studies on the biosynthesis and biodegradation of glycosaminoglycanes, performed on the brain and cerebellum of mice in a developing stage, by labelling with (35 S) and evaluation of the label 24 hours after the injection, have shown that the degree of biosynthesis of condroitin sulphate and heparan sulphate can be correlated temporally, but is different from the one of Dermatan sulphate (T. Burkart and U.N. Wiesmann, Dev. Biol. 1987, 120(2), 447).

In addition to the studies on the biosynthetic and metabolic processes of single cerebral glycosaminoglycanes in relation with the degree of maturation of the central nervous tissue, studies have also been performed on the variation of the structure of same, which can be correlated with the physiological state of the nervous tissue.

Studies in this connection have been performed on condroitin-4-sulphate and condroitin-6-sulphate proteoglycane utilising a specific antibody and detecting by electronic immunomicroscopy the localization of the proteoglycane itself in the mature brain and in the one in development (D.A. Aquino, R.U. Margolis and R.K. Margolis, J.Cell.Biol., 1984, 99 1117).

The data shown, together with the demonstration that in certain pathological events, which have an influence on the functionality of the cerebral nervous cell, quantitative variations of the glycosaminoglycane sulphates can be detected, suggest an involvment of the same in the morphogenesis and differentiations of the central nervous tissue (D.B. Wilson, J. Craniofac, Genet. Dev.Biol., 1985, 5(4), 363; K.E. Wisniewski, F.J. Kieras, J.H. French, G.E. Jr. Houck and P.L. Ramos, Ann, Neurol. 1984, 16(1), 40; A. Oohiza, P.Matsui, M. Matsuda and R. Shoji, J. Neurochem. 1976, 47(2), 588).

Although the exact interaction mechanism are not known, numerous hypotheses were made, connected in general with the physico-chemical characteristics of glycosaminoglycane sulphates.

It is accepted in general, for instance, that one of the most probable functions of the extra-cellular glycosaminoglycane sulphates, in particular of jaluronic acid, consists in making more easily penetrable the matrix through which the neuronal migration takes place, originating the differentiation which occurs during the brain development (D.A. Aquino, R.V. Margolis and R.K. Margolis, J. Cell, Biol., 1984, 99 1130).

An additional function, in accordance with the physico-chemical characteristics deriving from the polyanionic structure of these compounds, may be the interaction in the extra-cellular medium with the motion of ions, non-ionised compounds, water and large molecules thus influencing the transportation, the diffusion aliquot and the distribution of other solutes in the solutions and/or functioning as sieves or molecular barriers.

(B.M. Rice, G.A. Gerhardt, P.M. Hierl. G. Nagy and R.N. Adams, Neuroscience, 1985, 15(3) 891 P.A. Knepper and D.G. McLone, Pediatric NeuroSci., 1985-86 (4-5), 240).

A typical example of such a mechanism is given by the demonstration that the proteo-heparan sulphate, (N.C. Inestrosa and A. Perelman, TIPS, 1989, 10, 325), present on the cellular surface, is necessary for externalizing at the neuronal cellular surface level the asymmetrical forms of acetyl-cholinesterase.

Such a mechanism was observed in studies "in vitro" on rat pheochromocytoma PC 12 cells.

In a stable variant of these cells lacking proteo-heparan sulphate at the cellular surface level an atypic distribution of asymmetric acetylcholinesterase was noticed (normally, in the normal PC 12 cells the asymmetric acetylcholinesterase is essentially located externally to the cell surface, while in mutant cells the enzyme is found in the intracellular compartment).

Finally, the fact that the interaction between the various extracellular components is necessary for the coordination of the various processes activated during morphogenesis and differentiation, together with the demonstration of the disappearance of condroitin sulphate proteoglycane from the extra cellular region of the immature brain and its appearance as a cytoplasmatic component in the adult individual, is an indication that glycosaminoglycane sulphates may be involved in this very stage of the development of the central nervous system, and that the removal of condroitin sulphate proteoglycane from the extra cellular region may allow the activation of the "cell-to-cell" adhesion processes (D.A. Aquino, R.V. Margolis and R.K. Margolis, J. Cell. Biol., 1984, 99, 1130).

The above shows evidently that some proteoglycane sulphates take part in the growth and neuronal differentiation process.

The mechanisms involved were established in particular for jaluronic acid, condroitin sulphate and heparan sulphate.

On the contrary from the present state of the art no evidence exists of the physiological role of Dermatan sulphate at the central nervous system level.

Summary

We have now found that Dermatan sulphate, in its natural as well as in its low molecular weight form, possibly associated with heparin, takes part in the central nervous tissue formation, growth and differentiation processes and that, therefore, in view of these characteristics it can be used as an active substance for the preparation of pharmaceutical compositions suitable for the preventive and therapeutic treatment of cerebral aging, of infantile and senile demential forms, of syndroms correlated with an altered biochemistry of glycosaminoglycane sulphate, of the hypofunctionality of the central nervous system due to excessive use of drugs and to toxicodependency.

Said compositions comprise diluents and excipients normally employed in the pharmaceutical technique and may be administered both per os and parenterally.

Detailed description of the invention

The effects of Dermatan sulphate (intending with this term, in the course of the present description, both the natural form and the low molecular weight form) and of the Dermatan sulphate-heparin association in the processes of formation, growth and differentiation of the central nervous tissue, and the therapeutical indications, will be illustrated in the course of the following detailed description which refers to "in vitro" experiments performed on cells, and to "in vivo" experiments performed on mice from the gestation period up to the adult age.

The experiments were performed using: natural Dermatan sulphate obtained from proteoglycanes of animal vascolarized tissue, low molecular weight Dermatan sulphate, comprised in the range between 3,000 and 20,000 Daltons, both being of animal origin, and also both Dermatan sulphate forms with addition of small amounts of heparin.

The "in vitro" experiments were carried out on PC 12 cells deriving from rat pheochromocytoma, able to differentiate, after NGF (Nerve Growth Factor) addition, with formation of cells showing neuronal characteristics, and on neuronal cell cultures.

In said experiments the effect of Dermatan sulphate, as such and after addition of heparin, on the degree of growth and cell differentiation was evaluated in comparison with standard control models activated with NGF.

The effects "in vivo" on the degree of neuronal maturation were studied in mouse fetus and on mouse at different ages, by determining the enzymatic and specific surface marker activity for the neuronal cell.

The possibility was also controlled for Dermatan sulphate to reach and surpass the hematoencephalic barrier, after administration to the mouse of the compound labelled with biotin.

It was found that Dermatan sulphate:
- performs "in vitro" the same effects of NGF, increasing the growth and neuronal differentiation in substitution of the NGF itself and demonstrating a direct mechanism of interaction on the morphogenesis of the central nervous tissue;
- accelerates "in vivo" the degree of neuronal maturation;
- is able to overcome the hematoencephalic barrier;
- Dermatan sulphate in its natural form and in its low molecular weight form has the same effects on the nervous system;
- Dermatan sulphate in association with small amounts of heparin has shown a higher efficiency with respect to the unassociated products, with a synergic effect.

The above mentioned characteristics allow the use of Dermatan sulphate, as such and in association with heparin, in the preparation of pharmaceutical compositions with the excipients and diluents normally

EP 0 450 508 A2

employed in the pharmaceutical technique, for the preventive and therapeutic treatment of cerebral aging, of infantile and senile demential forms, associated or not with pathological events of various nature, of syndromes correlated to an altered biochemistry of glycosaminoglycane sulphates, such as for example the oculocerebrorenal Lowe syndrome and the Hurler-Scheic syndrome, of the hypofunctionality and dismetabolism of the central nervous system due to excessive use of drugs and to toxicodependency.

The administration may be performed per os in doses of 100 to 1000 mg/day or parenterally in doses from 50 to 200 mg/day of Dermatan sulphate. The amount of associated heparin is comprised between 0.5 and 10% by weight on Dermatan sulphate.

This amount was found the best compromise between activity increase and absence of the typical heparin collateral effects.

Pharmacological experiments

For the pharmacological experimentation the following substances were employed: - natural Dermatan sulphate (DS) with a molecular weight of between 20,000 and 40,000 Daltons (prepared according to USP 4783447);
- Dermatan sulphate ($DS_I$) with an average molecular weight of 3,000 Daltons;
- Dermatan sulphate ($DS_{II}$) with an average molecular weight of 8,000 Daltons;
- Dermatan sulphate ($DS_{III}$) with an average molecular weight of 15,000 Daltons;
- natural Dermatan sulphate (DS) containing 3% heparin by weight;
- Dermatan sulphate ($DS_{II}$) containing 3% heparin by weight.

The $DS_I$, $DS_{II}$, $DS_{III}$ Dermatan sulphate was prepared by depolymerization of natural Dermatan sulphate by gamma ray treatment.

The characteristics of the various types of Dermatan sulphate employed in the experiments were as follows:
- sulphate/carboxyl ratio
  DS     1-1,2
  $DS_I$     1-1,3
  $DS_{II}$     1-1,3
  $DS_{III}$     1-1,3
- uronic acids:
  DS     32-25%
  $DS_I$     30-33%
  $DS_{II}$     30-33%
  $DS_{III}$     30-33%
- electrophoretic identification:
  DS     positive
  $DS_I$     positive
  $DS_{II}$     positive
  $DS_{III}$     positive

In the experiments the following effects were studied:
A) Effect of Dermatan sulphate and of the Dermatan sulphate-heparin association on PC 12 cells "in vitro";
B) Effect of Dermatan sulphate and of the association with heparin on neurone growth and differentiation "in vitro";
C) Effect of the administration of Dermatan sulphate and of the heparin association in the prenatal and postnatal neuronal maturation in mice;
D) Analysis of the bio-availability of Dermatan sulphate at the cerebral level in the mouse.

In the following points the experimental operations and the obtained results are described.

A) Effect of Dermatan sulphate and of the Dermatan sulphate-heparin association on PC 12 cells "in vitro"

The PC 12 cells derive from a rat pheochromocytoma and have the characteristics of differenting, in the culture, in the presence of NGF (Nerve Growth Factor), producing cells which show the typical neuron characteristics.

The PC 12 cells were cultivated in a culture medium consisting of:
- RPM I 1640 medium with glutamine;
- Fetal calf serum 5%;

4

- Colt serum 10%.

The culture was activated in a thermostat at 37°C, in the presence of 5% $CO_2$.

In the presence of NGF (25 $\mu$g/ml) numerous neuritic extensions in the culture cells are observed.

The addition to the culture of Dermatan sulphate (DS) and ($DS_{II}$) (0.5 mg/ml) and the addition of Dermatan sulphate (DS) and ($DS_{II}$) containing 3% heparin (0.5 mg/ml),in substitution of NGF, promote in a very significant way the neuritic growth and differentiation.

B) Effect of Dermatan sulphate and of its association with heparin on neurone growth and differentiation "in vitro"

The compartmental barrier culture method of neuronal cells was employed (R.B. Campenot, Proc. Nat. Acad. Sci. USA, 1977, 74, 4516).

The experiments were performed on neuronal cells from dorsal root ganglia (DRG) of 15-17 days rat embrion.

The cells were dissociated by treatment with trypsine and, after separation of this last by centrifuging, were resuspended in a culture medium.

The cells were inseminated in the internal compartment of the double chambered Campenot system which allows to visualize and quantify the developing neuronal extensions.

On the plate a layer of collagen was introduced on which scratches were made, the cilynder was fastened on the plate center with silicone grease and methylcellulose, and the neuronal cells were inseminated in the cilynder.

The experiments were made under the following conditions:

a) cultures in the absence of NGF (controls);

b) cultures in the presence of NGF (1 $\mu$g/ml);

c) cultures in the presence of DS (0.5 mg/ml);

d) cultures in the presence of DS (1 mg/ml);

e) cultures in the presence of $DS_I$ (0.5 mg/ml);

f) cultures in the presence of $DS_{II}$ (0.5 mg/ml);

g) cultures in the presence of $DS_{III}$ (0.5 mg/ml);

h) cultures in the presence of DS + heparin (3%);

i) cultures in the presence of $DS_{II}$ + heparin (3%).

In the experiment conditions the neuritic extensions grow through the barrier and enter the chamber.

The results are reported in table 1 and refer to the number of neuritic extensions which have grown through the diffusion barrier and have developed in the external chamber.

## TABLE 1

| Number of neuritic extensions which have grown through the diffusion barrier and have developed in the external chamber (Neuron culture from DRG) | | | | | | | | | |
|---|---|---|---|---|---|---|---|---|---|
| **Growth factor** | **Concentration** | | **Days of culture** | | | | | | |
| | | | 7 | 8 | 9 | 13 | 15 | 16 | 19 | 23 |
| --- | --- | | 0 | 1 | 1 | 1 | 1 | 1 | 1 | 1 |
| NGF | 1 µg/ml | | 3 | 6 | 9 | 12 | 13 | 13 | 13 | 15 |
| DS | 0.5 mg/ml | | 2 | 4 | 4 | >40 | >40 | >40 | >40 | >40 |
| DS | 1 mg/ml | | 11 | 13 | 14 | >40 | >40 | >40 | >40 | >40 |
| $DS_I$ | 0.5 mg/ml | | 4 | 4 | 6 | >40 | >40 | >40 | >40 | >40 |
| $DS_{II}$ | 0.5 mg/ml | | 5 | 8 | 9 | >40 | >40 | >40 | >40 | >40 |
| $DS_{III}$ | 0.5 mg/ml | | 5 | 4 | 5 | >40 | >40 | >40 | >40 | >40 |
| DS + heparin 3% | 0.5 mg/ml | | 8 | 18 | 25 | >40 | >40 | >40 | >40 | >40 |
| $DS_{II}$ + heparin 3% | 0.5 mg/ml | | 6 | 15 | 23 | >40 | >40 | >40 | >40 | >40 |

Note 1: when the number of neuritic extension is higher than 40 it is no longer possible to count the growth.

Note 2: the same tests were made also with DS and $DS_{II}$ associations with 2% heparin and with $DS_I$ and $DS_{III}$ associations with 2% and with 3% heparin obtaining similar results.

C) Effect of the administration of Dermatan sulphate and of the Dermatan sulphate-heparin association on the neuronal maturation in pre- and post natal mouse

The degree of neuronal maturation was evaluated by determination, at the hypothalamic level, of the development of three enzymes capable of synthesizing neuro-transmitters (tyrosine hydrolase, choline acetyltransferase and glutamic acid decarboxylase) and of the neuronal cell surface markers specific for the bond with the tetanus toxin.

The differentiation of the surface neuronal markers and of the intraneuronal specific enzymes is correlated with the neuron maturation degree.

Dermatan sulphate and the Dermatan sulphate-heparin association were administered i.v. to the female mouse at the dose of 5 mg/Kg for 5 days prior to mating and for 7 additional days from the beginning of gestation.

The brains were taken at various pre natal and post natal age stages from the animals sacrified by decapitation, and were kept in phosphate buffer at -40 °C until the time of test.

The hypothalamus membranes for the test on the ability of binding the tetanus toxin, and the homogenates for the enzymatic activity test were prepared in accordance with Puymirat (J. Puymirat, A. Faivre-Baurnan, B.Bizzini and A. Tixier-Vidal, Develop. Brain Res., 1982, 3, 199).

The enzyme activity was evaluated on 10 μl of histic surnatant by the micromethod of Puymirat (J. Puymirat, F. Javoy-Agid, P. Gaspar, A. Ploska, A. Prochiantr and Y. Agid, J. Neurochem., 1979, 32, 449) for tyrosine hydrolase (TH); of Moskal and Basu (J. R. Moskal and S. Basu, Analyt. Biochem., 1975, 65, 449) for glutamic acid decarboxylase (GAD); of Rossier and of Fonnum (J. Rossier, A. Baurman and P. Benda, FEBS lett. 1973, 32, 231; F. Fonnum, J. Neurochem., 1975, 24, 407) for choline acetyl transferase (ChAT).

The capacity of binding the tetanus toxin was evaluated according to Puymirat and coll. (Develop. Brain Res. 1982,3, 199).

The results, expressed as percentage of the values which can be ascertained in the adult mouse, are reported in Table 2 and give evidence of the fact that the administration of DS, both natural and of low molecular weight, is capable of accelerating the growth and functionality of the central nervous cell.

TABLE 2 – Effect of the administration of Dermatan sulphate and of the Dermatan sulphate–heparin association on the brain development in mice

| Parameter | Compound | Dose | Enzymatic activity/binding capacity (% with respect to the adult) | | | | | | |
|---|---|---|---|---|---|---|---|---|---|
| | | | Gestation start | Pre-natal period (day) | | Birth | Post-natal period (day) | | |
| | | | | 14* | 18* | | 5* | 15* | 30* |
| Activity TH | Control | — | — | 25 | 41 | — | 76 | 81 | 100 |
| | DS | 5 mg/die i.v. x 12 gg | — | 38 | 54 | — | 87 | 98 | 100 |
| | DS I | " | — | 35 | 57 | — | 89 | 99 | 100 |
| | DS II | " | — | 35 | 55 | — | 90 | 98 | 100 |
| | DS + heparin 3% | " | — | 39 | 66 | — | 93 | 100 | 100 |
| | DS II + heparin 3% | " | — | 37 | 60 | — | 92 | 99 | 100 |
| Activity GAD | Control | — | — | 3 | 14 | — | 28 | 50 | 81 |
| | DS | " | — | 12 | 28 | — | 46 | 82 | 100 |
| | DS I | " | — | 15 | 32 | — | 45 | 78 | 100 |
| | DS II | " | — | 10 | 30 | — | 50 | 80 | 100 |
| | DS + heparin 3% | " | — | 15 | 39 | — | 72 | 90 | 100 |
| | DS II + heparin 3% | " | — | 15 | 37 | — | 68 | 85 | 100 |
| Activity ChAT | Control | — | — | 5 | 6 | — | 12 | 54 | 93 |
| | DS | " | — | 12 | 20 | — | 30 | 70 | 100 |
| | DS I | " | — | 10 | 22 | — | 34 | 72 | 100 |
| | DS II | " | — | 14 | 20 | — | 34 | 68 | 100 |
| | DS + heparin 3% | " | — | 18 | 30 | — | 50 | 90 | 100 |
| | DS II + heparin 3% | " | — | 20 | 32 | — | 52 | 95 | 100 |
| Capacity of tetanus toxin binding | Control | — | — | 22 | 42 | — | 67 | 90 | 100 |
| | DS | " | — | 35 | 50 | — | 84 | 100 | 100 |
| | DS I | " | — | 32 | 54 | — | 84 | 100 | 100 |
| | DS II | " | — | 32 | 50 | — | 85 | 100 | 100 |
| | DS + heparin 3% | " | — | 40 | 62 | — | 93 | 100 | 100 |
| | DS II + heparin 3% | " | — | 40 | 58 | — | 94 | 100 | 100 |

Tyrosin-hydrolase (TH), cholin-acetyltransferase (ChAT), glutamic acid decarboxylase (GAD) activity and binding capacity of the tetanus toxin, in the hypothalamus cells during the mouse brain development. Results expressed in percentage of the normal adult values (TH: 0.75 nmol/h.mg protein; GAD: 20.6 nmol/h.mg protein ; ChAT: 600 nmol/h mg protein; tetanus binding: 1600 cpm/μg protein). Each value represents the average of 5 experiments

D) Analysis of Dermatan sulphate bioavailability at a cerebral level

The pharmacokinetic study of compounds DS$_I$ and DS$_{II}$ was performed utilising for the administration to the animal the same compounds marked with biotin which, as known, is able to form a stable complex with Avidine (Ka = $10^{14}$ ).

The detection of a molecule marked with biotin may be made with peroxydase conjugated Avidine.

For the identification, the cyto-enzymatic technique was employed, which allows to precisely localize the Dermatan sulphate distribution at cell level.

The biotinilated Dermatan sulphate was administered to the mouse per os (5 mg/kg) and intramuscularly (1 mg/kg).

Groups of animals were sacrificed before (control) and at various times from the administration, extracting the brain which was frozen in liquid nitrogen and then passed to the cryomicrotome.

The peroxidase activity was blocked and then the detection of the biotinilated Dermatan sulphate/Avidine-peroxidase complex was performed by evaluation of the presence of peroxydase by reaction with diamminobenzidine.

The results are reported in Table 3 from which one can see that natural Dermatan sulphate and low molecular weight Dermatan sulphate, after parenteral and oral administration, overcome the hematoencephalic barrier, reaching the central nervous tissue.

## TABLE 3

Bioavailability at cerebral level of Dermatan sulphate conjugated with biotin after oral and intramuscle administration in mice

| Compound | way of administration | Dose | Presence of Dermatan sulphate in the cerebral cell | | | | |
|---|---|---|---|---|---|---|---|
| | | | 0 hours | 2 hours | 6 hours | 24 hours | 48 hours |
| DS | i.m. | 1 mg/kg | 0 | ± | ++ | + | ± |
| DS I | " | " | 0 | + | ++ | + | 0 |
| DS II | " | " | 0 | ± | ++ | + | ± |
| DS | os | 5 mg/kg | 0 | 0 | + | + | + |
| DS I | " | " | 0 | 0 | + | + | ± |
| DS II | " | " | 0 | 0 | + | + | ± |

± = little   + = presence   ++ = much

## Claims

1. The use of Dermatan sulphate and of Dermatan sulphate-heparin association for the preparation of pharmaceutical compositions suitable for the preventive and therapeutic treatment of cerebral aging, of infantile and senile demential forms, of syndroms correlated to altered biochemistry of glycosaminoglycane sulphates, of the hypofunctionality and dismetabolism of the central nervous system due to excessive use of drugs and to toxicodependency.

2. The use according to claim 1, characterized in that said Dermatan sulphate is of the natural type, with a molecular weight of between 20,000 and 40,000 Daltons.

3. The use according to claim 1, characterized in that said Dermatan sulphate is of the type of average molecular weight of 3,000 Daltons.

4. The use according to claim 1, characterized in that said Dermatan sulphate is of the type of average molecular weight of 8,000 Daltons.

5. The use according to claim 1, characterized in that said Dermatan sulphate is of the type of average molecular weight of 15,000 Daltons.

6. The use according to claim 1, characterized in that said Dermatan sulphate-heparin association has an heparin contents comprised between 0.5 and 10% by weight with respect to the Dermatan sulphate.

7. Pharmaceutical compositions comprising as an active substance Dermatan sulphate of natural type with a molecular weight of between 20,000 and 40,000 Daltons, in admixture with the excipients and diluents normally used in the pharmaceutical technique, and suitable for the preventive and therapeutic treatment of cerebral aging, of infantile and senile demential forms of syndrome correlated with an altered biochemistry of glycosaminoglycane sulphates, of the hypofunctionality and of the dismetabolism of the central nervous system due to an excessive use of drugs and to toxicodependency.

8. Pharmaceutical compositions comprising as an active substance Dermatan sulphate of the type of average molecular weight between 3,000 and 15,000 Daltons in admixture with the excipients and diluents normally used in the pharmaceutical technique, and suitable for the preventive and therapeutic treatment of cerebral aging, of infantile and senile demential forms, of syndrome correlated with an altered biochemistry of glycosaminoglycane sulphates, of the hypofunctionality and of the dismetabolism of the central nervous system due to an excessive use of drugs and to toxicodependency.

9. Pharmaceutical compositions according to claims 7 and 8, characterized in that said Dermatan sulphate is associated with heparin in an amount of between 0.5 and 10% by weight with respect to the Dermatan sulphate.

10. Pharmaceutical compositions comprising as an active substance Dermatan sulphate of natural type of a molecular weight of between 20,000 and 40,000 Daltons in admixture with the excipients and diluents normally employed in the pharmaceutical technique, and suitable for the preventive and therapeutic treatment of cerebral aging, of infantile and senile demential forms of syndrome correlated with an altered biochemistry of glycosaminoglycane sulphates, of the hypofunctionality and of the dismetabolism of the central nervous system due to an excessive use of drugs and to toxicodependency, to be administered per os at the dose of between 100 and 1000 mg/day of Dermatan sulphate.

11. Pharmaceutical compositions comprising as an active substance Dermatan sulphate of natural type of a molecular weight of between 20,000 and 40,000 Daltons in admixture with the excipients and diluents normally employed in the pharmaceutical technique, and suitable for the preventive and therapeutic treatment of cerebral aging, of infantile and senile demential forms of syndrome correlated with an altered biochemistry of glycosaminoglycane sulphates, of the hypofunctionality and of the dismetabolism of the central nervous system due to an excessive use of drugs and to toxicodependency, to be administered parenterally at the dose of between 50 and 200 mg/day of Dermatan sulphate.

12. Pharmaceutical compositions according to claim 10 and 11, characterized in that said Dermatan sulphate is associated with heparin in an amount of between 0.5 and 10% by weight with respect to the Dermatan sulphate.

13. Pharmaceutical compositions comprising as an active substance Dermatan sulphate of the type of average molecular weight between 3,000 and 15,000 Daltons in admixture with the excipients and diluents normally employed in the pharmaceutical technique and suitable for the preventive and therapeutic treatment of cerebral aging, of infantile and senile demential forms of syndrome correlated with an altered biochemistry of glycosaminoglycane sulphates, of the hypofuncitionality and of the dismetabolism of the central nervous system due to an excessive use of drugs and to toxicodependency, to be administered per os at the dose of between 100 and 1000 mg/day of Dermatan sulphate.

14. Pharmaceutical compositions comprising as an active substance Dermatan sulphate of the type of average molecular weight between 3,000 and 15,000 Daltons in admixture with the excipients and diluents normally employed in the pharmaceutical technique and suitable for the preventive and therapeutic treatment of cerebral aging, of infantile and senile demential forms, of syndrome correlated with an altered biochemistry of glycosaminoglycane sulphates, of the hypofunctionality and of the dismetabolism of the central nervous system due to an excessive use of drugs and to toxicodependency, to be administered parenterally at the dose of between 50 and to 200 mg/day of Dermatan sulphate.

15. Pharmaceutical composition according to claims 13 and 14, characterized in that said Dermatan sulphate is associated with heparin in an amount of between 0.5 and 10% with respect to the Dermatan sulphate.